(19) Europäisches Patentamt / European Patent Office / Office européen des brevets

(11) **EP 4 304 455 B1**

(12) **EUROPEAN PATENT SPECIFICATION**

(45) Date of publication and mention
of the grant of the patent:
**05.11.2025 Bulletin 2025/45**

(21) Application number: **22720226.4**

(22) Date of filing: **13.04.2022**

(51) International Patent Classification (IPC):
*A61B 5/00* (2006.01)          *A61B 5/1455* (2006.01)
*A61B 5/145* (2006.01)        *A61B 5/024* (2006.01)

(52) Cooperative Patent Classification (CPC):
**A61B 5/0075; A61B 5/02438; A61B 5/14546;
A61B 5/14553; A61B 5/725;** A61B 5/6814;
A61B 5/6826

(86) International application number:
**PCT/US2022/024568**

(87) International publication number:
**WO 2022/225762 (27.10.2022 Gazette 2022/43)**

(54) **NIRS/TISSUE OXIMETRY BASED METHOD TO MEASURE ARTERIAL BLOOD OXYGEN SATURATION FROM PULSATILE HEMOGLOBIN WAVEFORMS**

NIRS/GEWEBEOXIMETRIEBASIERTES VERFAHREN ZUR MESSUNG DER ARTERIELLEN BLUTSAUERSTOFFSÄTTIGUNG AUS PULSIERENDEN HÄMOGLOBINWELLENFORMEN

PROCÉDÉ À BASE DE NIRS/OXYMÉTRIE DE TISSU POUR MESURER LA SATURATION EN OXYGÈNE DU SANG ARTÉRIEL DE FORMES D'ONDE D'HÉMOGLOBINE PULSATILE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.04.2021 US 202163178120 P**

(43) Date of publication of application:
**17.01.2024 Bulletin 2024/03**

(73) Proprietor: **Becton, Dickinson and Company
Franklin Lakes, NJ 07417-1880 (US)**

(72) Inventor: **BENNI, Paul, B.
Irvine, CA 92614 (US)**

(74) Representative: **Venner, Julia Ann et al
Kilburn & Strode LLP
Lacon London
84 Theobalds Road
London WC1X 8NL (GB)**

(56) References cited:
**US-A1- 2008 300 474      US-A1- 2012 108 927
US-A1- 2015 208 950      US-A1- 2016 310 053
US-A1- 2018 140 237      US-A1- 2019 374 140**

**US-B1- 8 320 981      US-B2- 10 485 462
US-B2- 9 848 808**

• **UMAR LAZUARDI ET AL: "Design of Pulse Oximetry Based on Photoplethysmography and Beat Rate Signal Using DS-100 Probe Sensor for SpO2 Measurement", 2018 3RD INTERNATIONAL SEMINAR ON SENSORS, INSTRUMENTATION, MEASUREMENT AND METROLOGY (ISSIMM), IEEE, 4 December 2018 (2018-12-04), pages 25 - 29, XP033556453, [retrieved on 20190531], DOI: 10.1109/ISSIMM.2018.8727725**

• **NAM BUI ET AL: "PhO2", EMBEDDED NETWORK SENSOR SYSTEMS, ACM, 2 PENN PLAZA, SUITE 701NEW YORKNY10121-0701USA, 6 November 2017 (2017-11-06), pages 1 - 14, XP058396643, ISBN: 978-1-4503-5459-2, DOI: 10.1145/ 3131672.3131696**

• **ABAY TOMAS YSEHAK ET AL: "Reflectance Photoplethysmography as Noninvasive Monitoring of Tissue Blood Perfusion", IEEE TRANSACTIONS ON BIOMEDICAL ENGINEERING, IEEE, USA, vol. 62, no. 9, 1 September 2015 (2015-09-01), pages 2187 - 2195, XP011666604, ISSN: 0018-9294, [retrieved on 20150818], DOI: 10.1109/TBME.2015.2417863**

- EKHARE ABHISHEK SAKHARAM ET AL: "Design and Development of Low Unit Cost and Longer Battery Life Wireless Pulse Oximetry System", 2014 FOURTH INTERNATIONAL CONFERENCE ON ADVANCES IN COMPUTING AND COMMUNICATIONS, IEEE, 27 August 2014 (2014-08-27), pages 278 - 285, XP032652138, [retrieved on 20140919], DOI: 10.1109/ICACC.2014.73
- PANDEY RAJEEV KUMAR ET AL: "External temperature sensor assisted a new low power photoplethysmography readout system for accurate measurement of the bio-signs", MICROSYSTEM TECHNOLOGIES, BERLIN, DE, vol. 27, no. 6, 27 November 2020 (2020-11-27), pages 2315 - 2343, XP037471560, ISSN: 0946-7076, [retrieved on 20201127], DOI: 10.1007/S00542-020-05106-Y
- ASHOKA REDDY K ET AL: "A novel method for the measurement of oxygen saturation in arterial blood", 2011 IEEE INTERNATIONAL INSTRUMENTATION AND MEASUREMENT TECHNOLOGY CONFERENCE (I2MTC 2011) : HANGZHOU, CHINA, 10 - 12 MAY 2011, IEEE, PISCATAWAY, NJ, 10 May 2011 (2011-05-10), pages 1 - 5, XP031957381, ISBN: 978-1-4244-7933-7, DOI: 10.1109/IMTC.2011.5944066
- OHAD YOSSEF HAY ET AL: "Pulse Oximetry with Two Infrared Wavelengths without Calibration in Extracted Arterial Blood", SENSORS, vol. 18, no. 10, 15 October 2018 (2018-10-15), pages 3457, XP055699529, DOI: 10.3390/s18103457

**Description**

This application claims priority to U.S. Patent Application No. 63/178,120 filed April 22, 2021.

BACKGROUND

1. Technical Field

The present disclosure relates to methods and apparatus for non-invasively determining biological tissue oxygen parameters in general, and to non-invasive methods and apparatus for non-invasively determining arterial tissue oxygen saturation levels in particular.

2. Background Information

Near-infrared spectroscopy (NIRS) is a spectrophotometric method of continually monitoring tissue oxygenation. The NIRS method is based on the principle that light in the near-infrared range (700 to 1,000 nm) can pass easily through skin, bone, and other tissues where it encounters hemoglobin located mainly within micro-circulation passages (e.g., capillaries, arterioles, and venules). Hemoglobin exposed to light in the near infra-red range has specific absorption spectra that varies depending on its oxidation state (i.e., oxyhemoglobin ($HbO_2$) and deoxyhemoglobin (Hb) each act as a distinct chromophore). By using light sources that transmit near-infrared light at specific different wavelengths, and measuring changes in transmitted or reflected light attenuation, concentration changes of the oxyhemoglobin ($HbO_2$) and deoxyhemoglobin (Hb) can be monitored.

A NIRS oximetry system typically includes a processor in communication with one or more sensors, each sensor having at least one light source and at least one light detector. Light emitted from the light source enters the subject's tissue. The emitted light (i.e., photons) travels different paths through the tissue from the light source to the detectors due to the high light scattering characteristics of tissue (wavelength dependent) and is attenuated as it passes through the tissue. More specifically, the attenuation of certain wavelengths of light is attributable to the presence of $HbO_2$ and Hb. By measuring the light attenuation between the emitted light and the light sensed at the respective light detector, concentration changes of $HbO_2$ and Hb can be determined.

Some NIRS sensors are known to have a "near" light detector and a "far" light detector. The near detector is positioned closer to the light source than the far detector. The path of light sensed by the near detector is understood to differ from the path of light sensed by the far detector. Very often the near detector is positioned to detect light that has primarily passed through tissue (e.g., scalp and skull tissue) overlying the organ of interest (e.g., the brain), and the far detector is positioned to detect light that has primarily passed through both the overlying tissue and organ tissue disposed beneath the overlying tissue. Although the light path between the light source and the near light detector differs from the light path between the light source and the far light detector, both paths may be characterized as being "banana shaped" (e.g., substantially elliptical), each having a different path length and depth of penetration. A general rule in NIRS sensor design is that the mean photon tissue interrogation depth of the banana shaped light path is one half (½) the distance between the sensor light source and respective detector.

Because NIRS technology mainly interrogates the microvasculature of tissue, which includes arterioles, venules, and capillaries, a NIRS tissue oxygen saturation ($StO_2$) measurement is made on a mixture of both venous and arterial blood. The mean ratio of this mixture for brain has been estimated to be about 70% venous to 30% arterial blood volume (e.g., Ito et al., Arterial fraction of cerebral blood volume in humans measured by positron emission tomography, Ann Nucl Med 2001; Apr. 15(2):111-6). Under prior art teachings, the same mean ratio of 70% venous to 30% arterial blood volume is typically applied to all regions of the body (e.g., Pang CC, Measurement of body venous tone, J Pharmacol Toxicol Methods 2000; 44(2):341-60). Therefore, to validate NIRS, oxygenation measurements of both venous and arterial blood need to be considered from the venous output and arterial input of a target organ.

An organ receives oxygenated blood from one or more arteries. After the tissue of the organ extracts oxygen from the arterial blood for metabolism in the capillary bed, blood returns to the heart by one or more veins. Depending on the organ's metabolism level, the hemoglobin oxygen saturation of venous blood is typically low and can vary depending on the particular organ. Smaller veins drain into larger veins and then into the superior vena cava (SVC) or inferior vena cava (IVC) into the heart's right atrium (RA) and right ventricle (RV). Blood drawn from these locations can be called "central" venous (CV) blood, which will have an oxygen saturation somewhere in between the highest and lowest individual venous oxygen saturations of the organs. Besides the usual organs, skin and skeletal muscle metabolize oxygen significantly as well, because of the skin's large surface area and skeletal muscle's large mass relative to the rest of the body. For the brain, arterial blood supply is primarily from the carotid arteries and the primary venous drainage is by the internal jugular vein / jugular bulb. The liver (or hepatic) venous oxygen saturation tends to be lower than the intestine/mesenteric portal vein oxygen saturation since the portal vein supplies low oxygenated blood to the liver. As stated above, a NIRS tissue oxygen

saturation ($StO_2$) measurement performed as described above reflects a mixture of both venous and arterial blood, the ratio of venous and arterial blood is estimated under prior art devices, and the ratio can actually vary substantially from the estimated ratio.

What is needed is a non-invasive method and apparatus that can measure the tissue arterial blood saturation rather than rely on an estimation. US 2019/374140 A1 discloses calculation of SpO2 on the basis of a ratio of ratios of pulsatile to non-pulsatile components at two different wavelengths. ASHOKA REDDY K ET AL: "A novel method for the measurement of oxygen saturation in arterial blood",2011 IEEE INTERNATIONAL INSTRUMENTATION AND MEASUREMENT TECHNOLOGY CONFERENCE (I2MTC 2011) : HANGZHOU, CHINA, 10 - 12 MAY 2011, IEEE, PISCATAWAY, NJ, 10 May 2011 (2011-05-10), pages 1-5, XP031957381,DOI: 10.1109/ IMTC.2011.5944066ISBN: 978-1-4244-7933-7 discloses SpO2 calculation on the basis of peak-to-peak values of of red and IR PPG signals, taking into account attenuation characteristics of reduced and oxyhemoglobin (Hb and HbO).

SUMMARY

According to the present disclosure, a method as disclosed by claim 1 is provided. Further embodiments are defined by claims 2-5.

According to another aspect of the present disclosure, an apparatus according to claim 6 is provided. Further embodiments are defined by claims 7-10.

According to another aspect of the present disclosure, a non-transitory computer-readable medium according to claim 11 is provided.

BRIEF DESCRIPTION OF THE DRAWINGS

FIG. 1 is a diagrammatic of an embodiment of a present disclosure NIRS sensing system with sensor transducers applied to a subject's forehead.
FIG. 2 is a diagrammatic illustration of a sensor transducer in communication with a tissue surface disposed above a target organ (e.g., a brain).
FIG. 3A is a diagrammatic planar view of a sensor transducer having a light source, a near light detector, and a far light detector.
FIG. 3B is a diagrammatic side view of the sensor transducer shown in FIG. 3A.
FIG. 4 is a diagrammatic illustration of a sensor transducer in communication with a tissue surface (e.g., a digit).

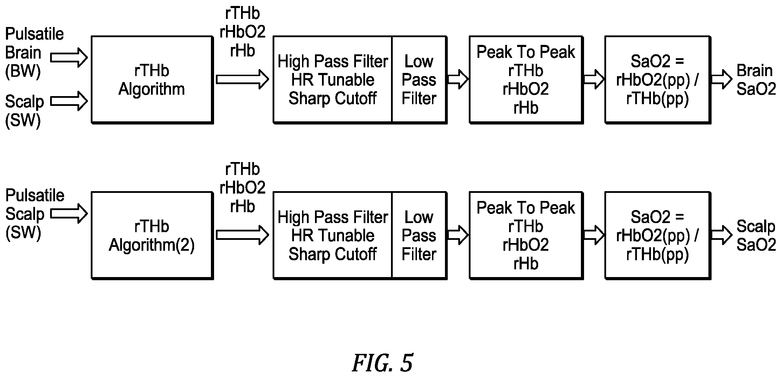

FIG. 5 is a diagrammatic illustration of signal processing used in some embodiments of the present disclosure.
FIG. 6 is a flowchart illustrating functions of an embodiment of the present disclosure.
FIG. 7 is a flowchart illustrating functions of an embodiment of the present disclosure.

DISCLOSURE OF THE INVENTION

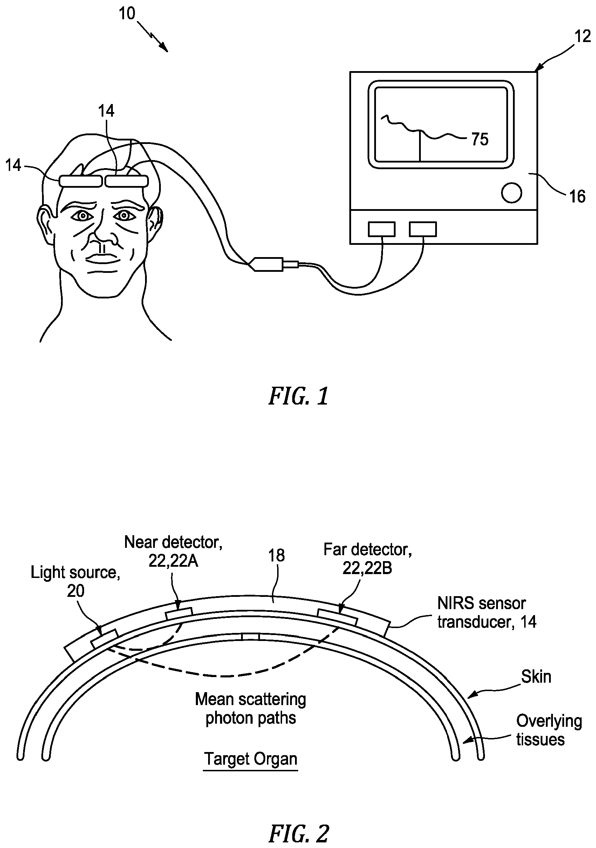

Referring to FIG. 1, the present method of and apparatus 10 for non-invasively determining an arterial tissue oxygen saturation includes a near infrared spectrophotometric (NIRS) tissue sensing device 12 (which may be referred to herein as a "NIRS oximeter 12") that includes one or more sensor transducers 14 and a controller 16. Each sensor transducer 14 is configured to selectively transmit light signals into the tissue (i.e., a tissue body) of a subject and to sense the transmitted light signals once they have passed through the tissue via transmittance or reflectance. The present disclosure is primarily described herein as including a NIRS cerebral oximeter to facilitate the description. The present disclosure is not, however, limited to including a NIRS cerebral oximeter. Oximeters configured to sense other tissue regions may be used alternatively; e.g., pulse oximeters configured to non-invasively sense digit or earlobe tissue. The NIRS oximeter 12 diagrammatically shown in FIG. 1 is a NIRS cerebral oximeter that includes a pair of sensor transducers 14 attached to a subject's forehead; e.g., to sense the right and left hemisphere of the subject's brain.

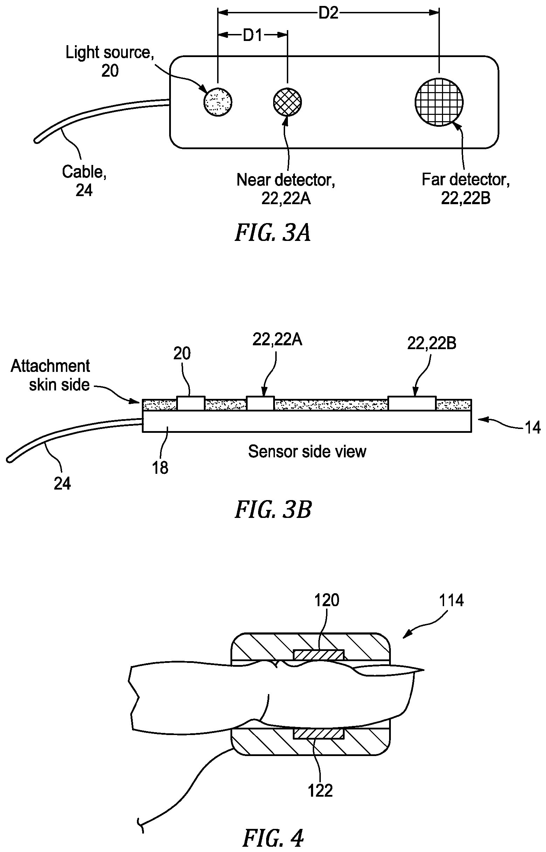

An example of an acceptable NIRS sensor transducer 14 is diagrammatically shown in FIG. 2. The sensor transducer 14, which may include a flexible body 18 that can be attached directly to a subject's tissue surface, includes one or more light sources 20 and one or more light detectors 22 mounted to or within the body 18. The light detectors may be described as a "near" detector 22A and a "far" detector 22B, where the terms "near" and "far" indicate the relative distances from the light source 20. FIGS. 3A and 3B provide a diagrammatic planar view and a side view of a sensor transducer 14 having a light source 20, a near light detector 22A, and a far light detector 22B. The near light detector 22A is separated from the light source 20 by a distance "D1" and the far light detector 22B is separated from the light source 20 by a distance "D2", where D2 is greater than D1 (D2 > D1). A disposable adhesive envelope or pad may be used to mount the sensor transducer 14 easily and securely to the subject's tissue surface. The light sources 20 may be light emitting diodes ("LEDs") that emit light at a narrow spectral bandwidth at predetermined wavelengths. The present disclosure is not, however, limited to using LEDs as a light source 20. A connector cable 24 may be used to connect the sensor transducer 14 components (e.g., lights source 20, light detectors 22, etc.) of each sensor transducer 14 directly or indirectly to the controller 16. The light detectors 22 are configured to sense light and produce signals representative of the sensed light. A non-limiting example of a light detector 22 type that can be used is a photodiode. Non-limiting examples of acceptable NIRS sensor transducers 14 are described in U.S. Patent Publication No. 2014/0171761 and U.S. Pat. No. 8,428,674. The above described NIRS sensor transducer 14 is a non-limiting example of a sensor transducer that may be used with the present disclosure. As stated herein, embodiments of the present disclosure may utilize NIRS oximeter 12 configurations other than a NIRS cerebral oximeter; e.g., a pulse oximeter. For those embodiments that use light transmitted through a digit or other tissue appendage, a NIRS sensor transducer 114 may have a finger mounting configuration (e.g., see FIG. 4) that includes a cuff configured to position a light source 120 on one side of the subject's digit and a light detector 122 on the opposite side of the subject's digit. Light emitted from the light source 120 passes through the digit via transmittance and is sensed by the light detector 122.

The controller 16 is in communication with at least some of the components within each NIRS sensor transducer 14 (e.g., the light source(s) 20, light detector(s) 22, etc.) to control and/or receive signals therefrom to perform the functions described herein. The controller 16 may include any type of computing device, computational circuit, processor(s), CPU, computer, or the like capable of executing a series of instructions that are stored in memory. The instructions may include an operating system, and/or executable software modules such as program files, system data, buffers, drivers, utilities, and the like. The executable instructions may apply to any functionality described herein to enable the NIRS oximeter 12 to accomplish the same algorithmically and/or coordination of device components. The controller 16 may include a single memory device or a plurality of memory devices. The present disclosure is not limited to any particular type of non-transitory memory device, and may include read-only memory, random access memory, volatile memory, nonvolatile memory, static memory, dynamic memory, flash memory, cache memory, and/or any device that stores digital information. The controller 16 may include, or may be in communication with, an input device that enables a user to enter data and/or instructions, and may include, or be in communication with, an output device configured, for example to display information (e.g., a visual display or a printer), or to transfer data, etc. Communications between the controller 16 and the NIRS oximeter 12 and its components may be via a hardwire connection or via a wireless connection. A person of skill in the art will recognize that portions of the controller 16 may assume various forms (e.g., digital signal processor, analog device, etc.) capable of performing the functions described herein.

It is known that near-infrared light passes through tissue readily and is absorbed by certain biological molecules disposed within the tissue. Near-infrared spectroscopy (NIRS) detects oxygenation changes in biological tissue (e.g., brain, muscle, or other organs) mainly at the microcirculation level (capillaries, arterioles, and venules) based on different absorption characteristics of certain chromophores (e.g., $HbO_2$, Hb) in the near-infrared spectrum (660 - 1000 nm). Average light penetration into the tissue is about 2-3 cm with sub-second time resolution. Relative changes of the concentrations of the chromophores (e.g., $HbO_2$, Hb) can be quantified by using the modified Beer-Lambert Law, which quantifies optical attenuation in a highly scattering medium like biological tissue. An example of a modified Beer-Lambert law is as follows:

$$A_\lambda = -log\,(\,I\,/\,I_o\,)_\lambda = \alpha_\lambda * C * d * B \;+\; G \qquad \text{(Equation 1)}$$

where A is the optical attenuation in tissue at wavelength $\lambda$ (units: optical density or "OD"); $I_o$ is the incident light intensity (units: W/cm$^2$); $I$ is the detected light intensity; $\alpha_\lambda$ is the wavelength dependent absorption coefficient of the chromophore (units: OD * cm$^{-1}$ * $\mu$M$^{-1}$); C is the concentration of chromophore (units: $\mu$M); $d$ is the light source 20 to detector distance (units: cm); B is the light scattering differential pathlength factor (unit-less); and $G$ is a factor relating to tissue geometry and scattering of light (units: OD).

Absolute measurement of chromophore concentration is very difficult because G is unknown. However, over a reasonable measuring period of several hours to days, G remains constant, allowing for the possibility of measuring relative changes of the respective chromophore from a zero reference baseline. Thus, if time $t_2$ is an arbitrary time after the start of the optical measurement at $t_1$ (baseline), differential attenuation $\Delta A$ can be calculated, canceling out the variables G and $I_o$, providing that they remain constant. The objective is to determine change in chromophore (e.g., Hb, $HbO_2$) concentration between time $t_1$ and $t_2$ (e.g., $\Delta C = C(t_2) - C(t_1)$) from $\Delta A$ based on the equation:

$$\Delta A = -log\,(\,I_2\,/\,I_1\,)_\lambda = \alpha_\lambda * \Delta C * d * B \qquad \text{(Equation 2)}$$

NIRS algorithms designed to calculate the relative changes of more than one chromophore may use a multivariate form of Equation 2. Equation 3, shown below is a non-limiting example of a multivariate form of Equation 2:

$$\begin{bmatrix} -log(I_2/I_1)_{\lambda 1}/(d * B_{\lambda 1}) \\ -log(I_2/I_1)_{\lambda 2}/(d * B_{\lambda 2}) \\ \vdots \\ -log(I_2/I_1)_{\lambda n}/(d * B_{\lambda n}) \end{bmatrix} = \begin{bmatrix} \alpha_{Hb\lambda 1} & \alpha_{HbO2\lambda 1} \\ \alpha_{Hb\lambda 2} & \alpha_{HbO2\lambda 2} \\ \vdots \\ \alpha_{Hb\lambda n} & \alpha_{HbO2\lambda n} \end{bmatrix} * \begin{bmatrix} \Delta Hb \\ \Delta HbO2 \\ \vdots \\ \vdots \end{bmatrix} \qquad \text{(Equation 3)}$$

where the left hand side of Equation 3 may be shown in abbreviated form as [$\Delta A/(d*B)$], the first term on the right side of Equation 3 may be shown in abbreviated form as [$\alpha$], and the second term on the right side of Equation 3 may be shown in abbreviated form as [$\Delta c$]; e.g., see Equations 4 and 5 below. To distinguish between and to compute relative changes in oxyhemoglobin ($\Delta HbO_2$) and deoxyhemoglobin ($\Delta Hb$), it is necessary to sense the tissue using a minimum of two different wavelengths. The units of $\Delta HbO_2$ and $\Delta Hb$ are in $\mu$moles per liter of tissue ($\mu M$) which is determined from a dimensional analysis of equation 1. The differential pathlength factor $B$ is wavelength dependent, leading to the exemplary expression:

$$B_{\lambda n} = B_{800} * k_{\lambda n}$$

The parameter $B_{800}$ is a user selected differential pathlength factor at 800 nm. The present disclosure is not limited to a differential pathlength factor at 800 nm, however. If the number of wavelengths used to sense the tissue is equal to the number of chromophores of interest, then Equation 3 can be solved in matrix form:

$$[\Delta c] = [\alpha]^{-1} * [\Delta A/(d * B)] \qquad \text{(Equation 4)}$$

where [$\alpha$]$^{-1}$ is the inverse matrix of [$\alpha$], and [$\Delta c$] can be solved by Cramer's Rule. If the number of wavelengths used to sense the tissue is greater than the number of chromophores of interest, a least square multilinear regression method may be used to solve [$\Delta c$]:

$$[\Delta c] = (\alpha^T * \alpha)^{-1} * \alpha^T * [\Delta A/(d * B)] \qquad \text{(Equation 5)}$$

where $\alpha^T$ is the transpose of $\alpha$, and $(\alpha^T * \alpha)^{-1}$ is the inverse matrix of the products of matrices $\alpha^T$ and $\alpha$. In theory, the greater the number of wavelengths used, the greater the reduction of errors in the solution of $\Delta c$. The summation of $\Delta HbO_2$ and $\Delta Hb$ give relative changes in total hemoglobin concentration ($\Delta TotalHb = \Delta HbO_2 + \Delta Hb$). The above-described methodology is a non-limiting example that can be used to determine tissue oxygenation parameters. Non-limiting alternative methodologies are disclosed in U.S. Patent Nos. 7,072,701; 8,396,526; 8,788,004; and 10,918,321.

As stated above, tissue oxygen parameters (e.g., rTHb, HbO$_2$, Hb) concentrations (or changes in concentrations) determined using the methodology described above include both venous and arterial contributions. Prior art methods of determining the venous and arterial contributions independent of one another rely on an estimation of the venous to arterial ratio (e.g., 70% venous / 30% arterial), which estimate may not be accurate for all types of tissue. The present disclosure provides a methodology for determining the portion of tissue oxygen saturation attributable to arterial blood flow (SaO$_2$) using signals at the respective wavelengths associated with particular tissue oxygen parameters (e.g., rTHb, HbO$_2$, Hb).

For example in the manner described above, a NIRS oximeter 12 may be used to sense a subject's cerebral tissue to determine tissue oxygen parameter concentrations (or changes in concentrations); e.g., a device having one or more NIRS sensor transducers 14, each transducer 14 having one or more light sources 20 and one or more light detectors 22 mounted to or within the body 18 as shown in FIG. 1. The light detectors 22 may include a near light detector 22A and a far light detector 22B as described above. The light sources 20 emit predetermined wavelengths of light that interrogate the subject tissue, and the light detectors 22A, 22B sense the emitted light some distance(s) from the light source 20 after such light has passed through the subject's tissue. The light detectors 22A, 22B produce signals representative of the light originally emitted by the light source 20 (at a plurality of predetermined different wavelengths) and subsequently sensed by the respective light detector 22A, 22B. Those signals are subsequently processed (e.g., in the manner described above) to determine parameter values representative of the change in concentration (or the absolute concentration) of the respective tissue oxygen parameters (e.g., rTHb, HbO$_2$, Hb); e.g., see Equation 3 above.

The aforesaid determined parameter values are representative of the tissue oxygen parameters within the interrogated tissue as a function of time. As indicated above, the sensed tissue will include a microvasculature that includes arterioles, venules, and capillaries, and will therefore include arterial blood flow. Over time, the blood volume present within the tissue will vary as a result of the arterial blood flow. Hence, the signals produced by the light detectors 22 during tissue sensing and/or the parameter values determined from those signals may be described as having an "AC" component (i.e., a variable component) and a "DC" component (i.e., a substantially constant component). Embodiments of the present disclosure utilize the AC component to determine tissue oxygen saturation attributable to arterial blood. Pulse oximeters, in contrast, are known to use both AC and DC signals from different wavelengths.

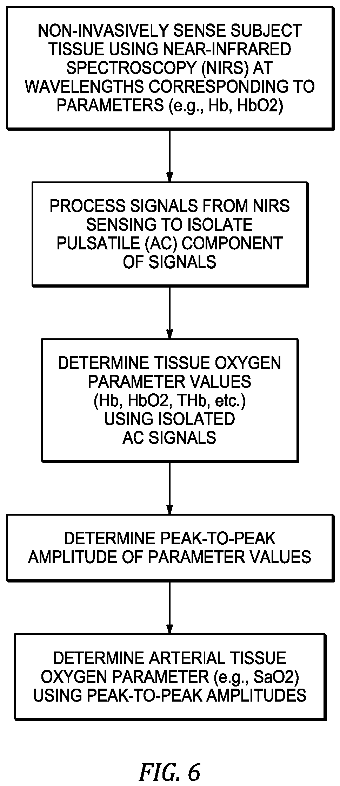

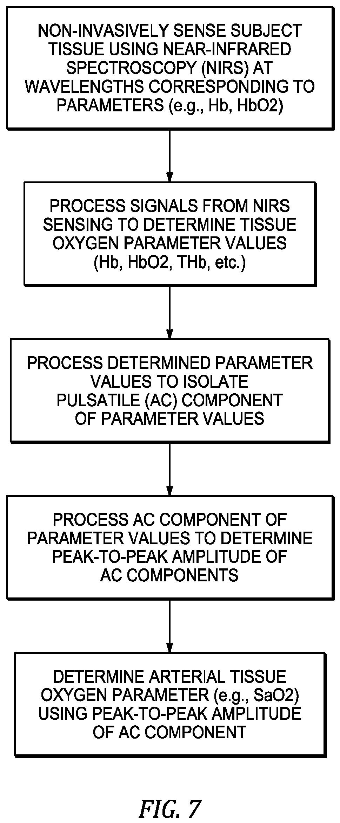

The AC component may be isolated by removing the DC component. Various different techniques can be used to isolate the AC component and the present disclosure is not limited to any particular technique. As an example, the signals produced by the light detectors 22 (which may be referred to as "raw signals") may be filtered using one or more high pass filters, low pass filters, band pass filters, or any combination thereof (see FIG. 6). For those embodiments that utilize a high pass filter, an acceptable example is a high pass filter having a sharp cutoff. In some embodiments, a tunable filter may be used; e.g., tunable to accommodate variations in heart rate. In those embodiments of the present disclosure that include a tunable filter, the NIRS oximeter 12 may include a device configured to determine the subject's heart rate or may be in communication with an independent device configured to determine the subject's heart rate. The aforesaid signal filtering may be performed within the controller 16 but is not required to be performed within the controller 16. The filtered signals representing the AC component may then be processed to determined tissue oxygen parameters (e.g., Hb, $HbO_2$, etc.) using an algorithmic approach such as shown in Equations 1-3. As an alternative (see FIG. 7), the aforesaid signals may be processed to produce parameter values and then those parameter values may be further processed to identify AC and DC components of the aforesaid parameter values and to isolate the AC component. Regardless of how it is isolated, the AC component may be characterized as being somewhat sinusoidal in shape with a peak-to-peak amplitude. The DC component may be discarded or may be utilized elsewhere.

As stated above, the present disclosure is configured to use the peak-to-peak amplitude of the AC component to determine the tissue arterial oxygen saturation (SaO2) level within the sensed tissue; e.g., via the stored instructions. More specifically, these embodiments of the present disclosure may utilize the peak-to-peak AC component attributable to $\Delta$TotalHb, $\Delta HbO_2$, and $\Delta$Hb (i.e., either the raw signals from the light detectors 22 attributable to $\Delta$TotalHb, $\Delta HbO_2$, and $\Delta$Hb, or the parameter values for $\Delta$TotalHb, $\Delta HbO_2$, and Hb) using the following equation (where "P-P" refers to peak to peak):

$$SaO2(NIRS) = \frac{(P-P\ amplitude\ \Delta Hb02)}{(P-P\ amplitude\ \Delta Total\ Hb)} * K \qquad \text{(Equation 6)}$$

A non-limiting alternative to Equation 6 is provided below:

$$SaO2(NIRS) = \left(1 - \frac{P-P\ amplitude\ \Delta Hb}{P-P\ amplitude\ \Delta Total\ Hb}\right) * K \qquad \text{(Equation 7)}$$

In Equations 6 and 7 the "P-P amplitude $\Delta$TotalHb" refers to the sum of the peak to peak amplitudes of Hb and $HbO_2$.

Equations 6 and 7 are examples of mathematical equations that may be used to determine $SaO_2$ values using the peak-to-peak AC components, and the present disclosure is not limited to these exemplary equations. The term "K" as used in Equations 6 and 7 is a corrective factor that may not be required (e.g., its value can be set to 1). The term K reflects the possibility that some amount of error or shift may occur when using the Beer-Lambert law (e.g., modified as indicated above) to determine the concentration of the respective chromophores based on their absorption of particular wavelengths of light.

The term K may be determined by a variety of different techniques, and therefore the present disclosure is not limited to any particular technique. An example of an acceptable technique involves producing a clinically sufficient amount of empirical $SaO_2$ data produced using a NIRS oximeter (i.e., "$SaO_2$ (NIRS)") and using an alternative sensing technology device such as a CO-oximeter (i.e., "$SaO_2$ (CO-ox REF)") on the same or substantially similar test tissue. The $SaO_2$ (CO-ox REF) data may be determined using the following equation:

$$SaO2(co - ox\ REF) = \frac{(P-P\ amplitude\ \Delta HbO2)}{(P-P\ amplitude\ \Delta Total\ Hb)} * K \qquad \text{(Equation 8)}$$

Equation 8 represents an example of an equation or technique that can be used to determine $SaO_2$ (CO-ox REF) and the present disclosure is not limited thereto. The respective $SaO_2$ data determined using the two techniques can subsequently be comparatively analyzed to ascertain the level of agreement between the data, and to determine a corrective factor (e.g., "K"), if necessary, to establish agreement there between; e.g., a "best fit". In some embodiments, a clinically sufficient amount of $SaO_2$ (NIRS) data and $SaO_2$ (CO-ox REF) data may be collected and organized using an X-Y scatterplot (e.g., with $SaO_2$ (CO-ox REF) on the X-axis and $SaO_2$ (NIRS) data on the Y-axis). The analysis of the data may include a regression technique to determine the relationship between the $SaO_2$ (CO-ox REF) data and the $SaO_2$ (NIRS) data and the term K representative of that relationship. A scatterplot analysis is a non-limiting example of a technique that may be used, and the present disclosure is not limited thereto. In some embodiments, the determination of a K value may take into consideration additional factors, such as the raw signals attributable to $\Delta$TotalHb, $\Delta HbO_2$, and $\Delta$Hb, light source 20 to light detector 22 distances, and the like; i.e.:

$$K = function(\Delta TotalHb, HbO_2, and\ or\ raw\ signals\ @diff\ \lambda s, LS$$
$$- LD\ separation, etc.)$$

From the above, it can be seen that the term K may be a constant. The term K may alternatively be an empirically derived algorithmic calibrating relationship; e.g., a linear equation, a polynomial equation, a non-linear equation, etc.

The isolated AC component may be quantitatively analyzed to determine the peak-to-peak amplitude using a variety of different techniques. For example, the AC component may be processed using a root mean square (RMS) filter to provide a value that is proportional to the peak-to-peak amplitude of the respective AC component; e.g., the AC components respectively associated with $\Delta TotalHb$, $\Delta HbO_2$, $\Delta Hb$, etc. When using an RMS filter, the peak-to-peak values of these respective AC components (as used in the determination of $SaO_2$) may be determined using the following equation:

$$P - P\ amplitude\ value =\ RMS * J \qquad\qquad (Equation\ 9)$$

where the term "J" is a constant or empirically determined corrective factor. As stated above, an RMS filter is an example of a means to determine a signal peak-to-peak amplitude value, and the present disclosure is not limited thereto.

As indicated above, the present disclosure may utilize different types of NIRS oximeters 12; e.g., cerebral oximeters, pulse oximeters, and any other NIRS sensing device operable to emit into a body of tissue and collect the emitted light reflected from the tissue, or the emitted light transmitted through the tissue, or any combination thereof. A non-limiting specific example of a NIRS cerebral oximeter is described above that utilizes sensor transducer 14 having a light source 20, one or more near light detectors 22A, and one or more far light detectors 22B (see FIGS. 2-3B). A benefit of the present disclosure using such a NIRS oximeter 12 stems from the different tissue bodies sensed as a result of collecting signal data using the spaced apart near and far light detectors 22A, 22B. As described above, the path of emitted light sensed by the near light detector 22A is understood to differ from the path of emitted light sensed by the far light detector 22B; i.e., the depth of tissue light interrogation sensed by the near light detector 22A is less than the depth of tissue light interrogation sensed by the far light detector 22B. Embodiments of the present disclosure that utilize a NIRS oximeter 12 with one or more NIRS sensor transducers 14 having near and far light detectors 22A, 22B can determine arterial tissue oxygen parameters for two different tissue bodies; e.g., arterial tissue oxygen parameters for a scalp and skull tissue region as well as arterial tissue oxygen parameters for a brain tissue region. The present disclosure is not limited to a NIRS cerebral oximeter, however. As indicated above, the present disclosure can utilize a finger mounted NIRS sensor transducer 114 (see FIG. 4) that includes a light source 120 disposed on one side of the subject's digit and a light detector 122 disposed on the opposite side of the subject's digit. Light emitted from the light source 120 passes through the digit via transmittance and is sensed by the light detector 122.

A benefit provided by some embodiments of the present disclosure is that they provide greater utility that currently available devices. Embodiments of the present disclosure that include a NIRS cerebral oximeter or the like that is configured to determine a tissue oxygen saturation value ($StO_2$) that includes contributions from both venous and arterial blood. The present disclosure can provide both $StO_2$ data and $SaO_2$ data; i.e., provide both $StO_2$ data and $SaO_2$ using a single device without the need to use a venous/arterial blood ratio estimation.

The present disclosure system 10 and method utilizing the AC component of light detector 22 signals and/or the AC component of determined tissue oxygen parameters (e.g., $HbO_2$, $Hb$) to determine $SaO_2$ data, are also understood to provide $SaO_2$ data with enhanced accuracy. Prior art systems for determining $SaO_2$ data (e.g., pulse oximeters) typically use light at a first wavelength (e.g., in the range of 660nm to 690nm) on one side of the hemoglobin isobestic point (i.e., the point where the light absorption of $HbO_2$ and $Hb$ are about the same ~ 805nm) and a second wavelength (e.g., in the range of 880nm to 940nm) on the opposite side of the isobestic point. As indicated above, the attenuation of light traveling through the tissue and the interrogation depth of that light is wavelength dependent at least in part due to pathlength factor "B". Hence, a tissue body interrogated by light at a first wavelength may not be the exact same tissue body interrogated by a light at a second, different wavelength, even if both wavelengths of light are emitted from and collected at the same light source 20 / light detector 22 combination. Embodiments of the present disclosure utilizing the AC component of signals and/or determined tissue oxygen parameter values can utilize wavelengths of light outside the aforesaid typical wavelength ranges (660-690 nm and 880-940 nm) on opposite sides of the isobestic point (~805nm) to determine $SaO_2$ data. More specifically, these embodiments may use wavelengths of light in the range of about 690nm - 810nm on one side of the isobestic point and may use wavelengths of light in the range of about 880nm - 810nm on the opposite side of the isobestic point. In a specific example, using the present disclosure, $SaO_2$ data can be produced using a first wavelength of light at about 770 nm and at about 870nm. As a result, the present disclosure permits arterial tissue oxygen

parameters (e.g., $SaO_2$, $HbO_2$, Hb) to be determined using wavelengths of light that are closer to one another than is the case using prior art methods. Hence, any differences in light attenuation and interrogation depth between a common light source 20 and light detector 22 attributable to wavelength dependent factors (e.g., pathlength factor "B") are decreased due to the closer proximity of the interrogating wavelengths; i.e., there is a greater likelihood that the tissue body interrogated by one wavelength more closely coincides with the tissue body interrogated by the other wavelength.

As indicated above, the functionality described herein may be implemented, for example, in hardware, software tangibly embodied in a computer-readable medium, firmware, or any combination thereof. In some embodiments, at least a portion of the functionality described herein may be implemented in one or more computer programs. Each such computer program may be implemented in a computer program product tangibly embodied in non-transitory signals in a machine-readable storage device for execution by a computer processor. Method steps of the present disclosure may be performed by a computer processor executing a program tangibly embodied on a computer-readable medium to perform functions of the present disclosure by operating on input and generating output. Each computer program within the scope of the present claims below may be implemented in any programming language, such as assembly language, machine language, a high-level procedural programming language, or an object-oriented programming language. The programming language may, for example, be a compiled or interpreted programming language.

While the principles of the disclosure have been described above in connection with specific apparatuses and methods, it is to be clearly understood that this description is made only by way of example and not as limitation on the scope of the disclosure. Specific details are given in the above description to provide a thorough understanding of the embodiments. However, it is understood that the embodiments may be practiced without these specific details.

It is noted that the embodiments may be described as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The singular forms "a," "an," and "the" refer to one or more than one, unless the context clearly dictates otherwise. For example, the term "comprising a specimen" includes single or plural specimens and is considered equivalent to the phrase "comprising at least one specimen." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements.

It is noted that various connections are set forth between elements in the present description and drawings. It is noted that these connections are general and, unless specified otherwise, may be direct or indirect and that this specification is not intended to be limiting in this respect. Any reference to attached, fixed, connected or the like may include permanent, removable, temporary, partial, full and/or any other possible attachment option.

As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

While various aspects, concepts and features of the disclosures may be described and illustrated herein as embodied in combination in the exemplary embodiments, these various aspects, concepts, and features may be used in many alternative embodiments, either individually or in various combinations and sub-combinations thereof. Unless expressly excluded herein all such combinations and sub-combinations are intended to be within the scope of the present disclosure. Still further, while various alternative embodiments as to the various aspects, concepts, and features of the disclosures--such as alternative materials, structures, configurations, methods, devices, and components, and so on--may be described herein, such descriptions are not intended to be a complete or exhaustive list of available alternative embodiments, whether presently known or later developed. Those skilled in the art may readily adopt one or more of the aspects, concepts, or features into additional embodiments and uses within the scope of the present application even if such embodiments are not expressly disclosed herein. For example, in the exemplary embodiments described above within the Detailed Description portion of the present specification, elements may be described as individual units and shown as independent of one another to facilitate the description. In alternative embodiments, such elements may be configured as combined elements.

Additionally, even though some features, concepts, or aspects of the disclosures may be described herein as being a preferred arrangement or method, such description is not intended to suggest that such feature is required or necessary unless expressly so stated. Still further, exemplary, or representative values and ranges may be included to assist in understanding the present application, however, such values and ranges are not to be construed in a limiting sense and are intended to be critical values or ranges only if so expressly stated.

**Claims**

1.  A computer-implemented method of non-invasively determining a tissue arterial oxygen saturation value of a tissue body, comprising:

    transmitting at least a first wavelength and a second wavelength of near-infrared light into a tissue body, the first wavelength different from the second wavelength;

    sensing the tissue body for the near-infrared light, and producing signals representative of the sensed near-infrared light, wherein the signals produced representative of the sensed near-infrared light include first signals representative of the first wavelength of near-infrared light, the first signals having an AC component and a DC component, and second signals representative of the second wavelength of near-infrared light, the second signals having an AC component and a DC component;

    processing the first signals to isolate an AC component of the first signals, wherein the step of processing the first signals to isolate the AC component of the first signals includes filtering the first signals to remove the DC component of the first signals;

    determining an amplitude of the AC component of the first signals, wherein the step of determining an amplitude of the AC component of the first signals includes determining a peak-to-peak amplitude of the AC component of the first signals;

    determining an AC component of a first tissue oxygen parameter using the signals, wherein the first tissue oxygen parameter is oxyhemoglobin ($HbO_2$), wherein the step of determining the AC component of the first tissue oxygen parameter of the tissue uses the determined peak-to-peak amplitude of the AC component of the first signals;

    processing the second signals to isolate an AC component of the second signals, wherein the step of processing the second signals to isolate the AC component of the second signals includes filtering the second signals to remove the DC component of the second signals;

    determining an amplitude of the AC component of the second signals, wherein the step of determining an amplitude of the AC component of the second signals includes determining a peak-to-peak amplitude of the AC component of the second signals;

    determining an AC component of a second tissue oxygen parameter using the signals, wherein the second tissue oxygen parameter is deoxyhemoglobin (Hb), wherein the step of determining the AC component of the second tissue oxygen parameter uses the determined peak-to-peak amplitude of the AC component of the second signals; and

    determining a tissue arterial oxygen saturation value of a tissue body using the determined AC component of the first tissue oxygen parameter and the determined AC component of the second tissue oxygen parameter, wherein the step of determining the tissue arterial oxygen saturation uses a ratio of the determined AC component of the first tissue oxygen parameter and a sum of the determined AC component of the first tissue oxygen parameter and the determined AC component of the second tissue oxygen parameter.

2.  The method of claim 1, wherein the step of determining a peak-to-peak amplitude of the AC component of the first signals includes filtering the AC component of the first signals to determine a value representative of the peak-to-peak amplitude of the AC component of the first signals; and
    wherein the step of determining a peak-to-peak amplitude of the AC component of the second signals includes filtering the AC component of the second signals to determine a value representative of the peak-to-peak amplitude of the AC component of the second signals.

3.  The method of claim 1, wherein the step of determining the AC component of the first tissue oxygen parameter includes determining a peak-to-peak amplitude of the AC component of the first tissue oxygen parameter; and
    wherein the step of determining the AC component of the second tissue oxygen parameter includes determining a peak-to-peak amplitude of the AC component of the second tissue oxygen parameter.

4.  The method of claim 1, wherein the at least said first wavelength and said second wavelength are transmitted using a light source from a sensor transducer having at least one near detector and at least one far detector, wherein the at least one near detector is located a first distance from the light source and the at least one far detector is located a second distance from the light source and the second distance is greater than the first distance; and
    wherein the sensing step utilizes the at least one near detector and the at least one far detector.

5.  The method of claim 1, wherein the transmitting step and the sensing step utilize a sensor transducer having a light source and a light detector, the sensor transducer configured to receive the tissue body in a manner such that the tissue body is disposed between the light source and the light detector and the transmitted near-infrared light is transmitted from the light source, through the tissue body in a direction toward the light detector.

6. An apparatus for non-invasively determining a tissue arterial oxygen saturation value of a tissue body, comprising:

at least one sensor transducer having a light source configured to produce at least a first wavelength and a second wavelength of near-infrared light, and at least one light detector configured to sense the at least said first wavelength and said second wavelength of near-infrared light; and
a controller in communication with the at least one sensor transducer, the controller including at least one processor and a memory device configured to store instructions, the stored instructions when executed cause the controller to:

control the light source to transmit at least a first wavelength and a second wavelength of near-infrared light into a tissue body, the first wavelength different from the second wavelength;
control the at least one light detector to sense the tissue body for the near-infrared light, and produce signals representative of the sensed near-infrared light, wherein the signals produced representative of the sensed near-infrared light include first signals representative of the first wavelength of near-infrared light, the first signals having an AC component and a DC component, and second signals representative of the second wavelength of near-infrared light, the second signals having an AC component and a DC component;
process the first signals to isolate an AC component of the first signals,

wherein the processing of the first signals to isolate the AC component of the first signals includes filtering the first signals to remove the DC component of the first signals;

determine an amplitude of the AC component of the first signals, wherein the determination of the amplitude of the AC component of the first signals includes determining a peak-to-peak amplitude of the AC component of the first signals;
determine an AC component of a first tissue oxygen parameter using the signals, wherein the first tissue oxygen parameter is oxyhemoglobin (HbO$_2$), wherein the determination of the AC component of the first tissue oxygen parameter uses the determined peak-to-peak amplitude of the AC component of the first signals;
process the second signals to isolate an AC component of the second signals, wherein the processing of the second signals to isolate the AC component of the second signals includes filtering the second signals to remove the DC component of the second signals;
determine an amplitude of the AC component of the second signals, wherein the determination of the amplitude of the AC component of the second signals includes determining a peak-to-peak amplitude of the AC component of the second signals;
determine an AC component of a second tissue oxygen parameter using the signals, wherein the second tissue oxygen parameter is deoxyhemoglobin (Hb), wherein the step of determining the AC component of the second tissue oxygen parameter uses the determined peak-to-peak amplitude of the AC component of the second signals; and
determine a tissue arterial oxygen saturation value of the tissue body using the determined AC component of the first tissue oxygen parameter and the determined AC component of the second tissue oxygen parameter, , wherein the step of determining the tissue arterial oxygen saturation uses a ratio of the determined AC component of the first tissue oxygen parameter and a sum of the determined AC component of the first tissue oxygen parameter and the determined AC component of the second tissue oxygen parameter.

7. The apparatus of claim 6, wherein the determination of the peak-to-peak amplitude of the AC component of the first signals includes filtering the AC component of the first signals to determine a value representative of the peak-to-peak amplitude of the AC component of the first signals; and
wherein the determination of the peak-to-peak amplitude of the AC component of the second signals includes filtering the AC component of the second signals to determine a value representative of the peak-to-peak amplitude of the AC component of the second signals.

8. The apparatus of claim 6, wherein the determination of the AC component of the first tissue oxygen parameter includes determining a peak-to-peak amplitude of the AC component of the first tissue oxygen parameter; and
wherein the determination of the AC component of the second tissue oxygen parameter of the tissue includes determining a peak-to-peak amplitude of the AC component of the second tissue oxygen parameter of the tissue.

9. The apparatus of claim 6, wherein the at least one detector of the at least one sensor transducer includes at least one near detector and at least one far detector, wherein the at least one near detector is located a first distance from the light source and the at least one far detector is located a second distance from the light source and the second distance is greater than the first distance.

10. The apparatus of claim 6, wherein the at least one sensor transducer is configured to receive the tissue body in a manner such that the tissue body is disposed between the light source and the light detector and the transmitted near-infrared light is transmitted from the light source, through the tissue body in a direction toward the light detector.

11. A non-transitory computer-readable medium containing computer program instructions, wherein the computer program instructions are executable by the at least one computer processor to perform a method of non-invasively determining a tissue arterial oxygen saturation value of a tissue body, the method comprising:

controlling a light source to transmit at least a first wavelength and a second wavelength of near-infrared light into a tissue body, the first wavelength different from the second wavelength;
controlling at least one light detector to sense the tissue body for the near-infrared light, and producing signals representative of the sensed near-infrared light, wherein the signals produced representative of the sensed near-infrared light include first signals representative of the first wavelength of near-infrared light, the first signals having an AC component and a DC component, and second signals representative of the second wavelength of near-infrared light, the second signals having an AC component and a DC component;
processing the first signals to isolate an AC component of the first signals, wherein the step of processing the first signals to isolate the AC component of the first signals includes filtering the first signals to remove the DC component of the first signals;
determining an amplitude of the AC component of the first signals, wherein the step of determining an amplitude of the AC component of the first signals includes determining a peak-to-peak amplitude of the AC component of the first signals;
determining an AC component of a first tissue oxygen parameter using the signals, wherein the first tissue oxygen parameter is oxyhemoglobin ($HbO_2$), wherein the step of determining the AC component of the first tissue oxygen parameter of the tissue uses the determined peak-to-peak amplitude of the AC component of the first signals;
processing the second signals to isolate an AC component of the second signals, wherein the step of processing the second signals to isolate the AC component of the second signals includes filtering the second signals to remove the DC component of the second signals;
determining an amplitude of the AC component of the second signals, wherein the step of determining an amplitude of the AC component of the second signals includes determining a peak-to-peak amplitude of the AC component of the second signals;
determining an AC component of a second tissue oxygen parameter using the signals, wherein the second tissue oxygen parameter is deoxyhemoglobin (Hb), wherein the step of determining the AC component of the second tissue oxygen parameter uses the determined peak-to-peak amplitude of the AC component of the second signals; and
determining a tissue arterial oxygen saturation value of a tissue body using the determined AC component of the first tissue oxygen parameter and the determined AC component of the second tissue oxygen parameter, wherein the step of determining the tissue arterial oxygen saturation uses a ratio of the determined AC component of the first tissue oxygen parameter and a sum of the determined AC component of the first tissue oxygen parameter and the determined AC component of the second tissue oxygen parameter.

**Patentansprüche**

1. Computerimplementiertes Verfahren zum nichtinvasiven Ermitteln eines Wertes der arteriellen Sauerstoffsättigung in Gewebe eines Gewebekörpers, umfassend:

Übertragen wenigstens einer ersten Wellenlänge und einer zweiten Wellenlänge eines Nah-Infrarot-Lichtes in einen Gewebekörper, wobei sich die erste Wellenlänge von der zweiten Wellenlänge unterscheidet,
Abtasten des Gewebekörpers für das Nah-Infrarot-Licht und Erzeugen von Signalen, die repräsentativ für das abgetastete Nah-Infrarot-Licht sind, wobei die erzeugten für das abgetastete Nah-Infrarot-Licht repräsentativen Signale erste Signale, die für die erste Wellenlänge des Nah-Infrarot-Lichtes repräsentativ sind und eine AC-Komponente und eine DC-Komponente aufweisen, und zweite Signale, die für die zweite Wellenlänge des Nah-Infrarot-Lichtes repräsentativ sind und eine AC-Komponente und eine DC-Komponente aufweisen, umfassen,

Verarbeiten der ersten Signale, um eine AC-Komponente der ersten Signale zu isolieren, wobei der Schritt des Verarbeitens der ersten Signale zum Isolieren der AC-Komponente der ersten Signale das Filtern der ersten Signale, um die DC-Komponente der ersten Signale zu entfernen, umfasst,

Ermitteln einer Amplitude der AC-Komponente der ersten Signale, wobei der Schritt des Ermittelns einer Amplitude der AC-Komponente der ersten Signale das Ermitteln eines Spitze-Tal-Wertes der AC-Komponente der ersten Signale umfasst,

Ermitteln einer AC-Komponente eines ersten Gewebesauerstoffparameters unter Verwendung der Signale, wobei es sich bei dem ersten Gewebesauerstoffparameter um Oxyhämoglobin ($HbO_2$) handelt, wobei der Schritt des Ermittelns der AC-Komponente des ersten Gewebesauerstoffparameters des Gewebes den ermittelten Spitze-Tal-Wert der AC-Komponente der ersten Signale verwendet,

Verarbeiten der zweiten Signale, um eine AC-Komponente der zweiten Signale zu isolieren, wobei der Schritt des Verarbeitens der zweiten Signale zum Isolieren der AC-Komponente der zweiten Signale das Filtern der zweiten Signale, um die DC-Komponente der zweiten Signale zu entfernen, umfasst,

Ermitteln einer Amplitude der AC-Komponente der zweiten Signale, wobei der Schritt des Ermittelns einer Amplitude der AC-Komponente der zweiten Signale das Ermitteln eines Spitze-Tal-Wertes der AC-Komponente der zweiten Signale umfasst,

Ermitteln einer AC-Komponente eines zweiten Gewebesauerstoffparameters unter Verwendung der Signale, wobei es sich bei dem zweiten Gewebesauerstoffparameter um Desoxyhämoglobin (Hb) handelt, wobei der Schritt des Ermittelns der AC-Komponente des zweiten Gewebesauerstoffparameters den ermittelten Spitze-Tal-Wert der AC-Komponente der zweiten Signale verwendet, und Ermitteln eines Wertes der arteriellen Sauerstoffsättigung in Gewebe eines Gewebekörpers unter Verwendung der ermittelten AC-Komponente des ersten Gewebesauerstoffparameters und der ermittelten AC-Komponente des zweiten Gewebesauerstoffparameters, wobei der Schritt des Ermittelns der arteriellen Sauerstoffsättigung in Gewebe ein Verhältnis der ermittelten AC-Komponente des ersten Gewebesauerstoffparameters zu einer Summe aus der ermittelten AC-Komponente des ersten Gewebesauerstoffparameters und der ermittelten AC-Komponente des zweiten Gewebesauerstoffparameters verwendet.

2. Verfahren nach Anspruch 1, wobei der Schritt des Ermittelns eines Spitze-Tal-Wertes der AC-Komponente der ersten Signale das Filtern der AC-Komponente der ersten Signale umfasst, um einen Wert zu ermitteln, der repräsentativ für den Spitze-Tal-Wert der AC-Komponente der ersten Signale ist, und wobei der Schritt des Ermittelns eines Spitze-Tal-Wertes der AC-Komponente der zweiten Signale das Filtern der AC-Komponente der zweiten Signale umfasst, um einen Wert zu ermitteln, der repräsentativ für den Spitze-Tal-Wert der AC-Komponente der zweiten Signale ist.

3. Verfahren nach Anspruch 1, wobei der Schritt des Ermittelns der AC-Komponente des ersten Gewebesauerstoffparameters das Ermitteln eines Spitze-Tal-Wertes der AC-Komponente des ersten Gewebesauerstoffparameters umfasst und wobei der Schritt des Ermittelns der AC-Komponente des zweiten Gewebesauerstoffparameters das Ermitteln eines Spitze-Tal-Werts der AC-Komponente des zweiten Gewebesauerstoffparameters umfasst.

4. Verfahren nach Anspruch 1, wobei die wenigstens erste Wellenlänge und zweite Wellenlänge unter Verwendung einer Lichtquelle von einem Sensorwandler, der wenigstens einen nahen Detektor und wenigstens einen entfernten Detektor aufweist, übertragen werden, wobei sich der wenigstens eine nahe Detektor in einem ersten Abstand zu der Lichtquelle befindet und sich der wenigstens eine entfernte Detektor in einem zweiten Abstand zu der Lichtquelle befindet und der zweite Abstand größer als der erste Abstand ist, und wobei der Abtastschritt den wenigstens einen nahen Detektor und den wenigstens einen entfernten Detektor nutzt.

5. Verfahren nach Anspruch 1, wobei der Übertragungsschritt und der Abtastschritt einen Sensorwandler nutzen, der eine Lichtquelle und einen Photodetektor aufweist, wobei der Sensorwandler dazu ausgelegt ist, den Gewebekörper auf eine solche Weise zu empfangen, dass der Gewebekörper zwischen der Lichtquelle und dem Photodetektor angeordnet ist und das übertragene Nah-Infrarot-Licht von der Lichtquelle durch den Gewebekörper in einer zu dem Photodetektor verlaufenden Richtung übertragen wird.

6. Einrichtung zum nichtinvasiven Ermitteln eines Wertes der arteriellen Sauerstoffsättigung in Gewebe eines Gewebekörpers, umfassend:

wenigstens einen Sensorwandler, der eine Lichtquelle, die dazu ausgelegt ist, wenigstens eine erste Wellenlänge und eine zweite Wellenlänge eines Nah-Infrarot-Lichtes zu erzeugen, und wenigstens einen Photodetektor, der dazu ausgelegt ist, die wenigstens erste Wellenlänge und zweite Wellenlänge des Nah-Infrarot-Lichtes abzutasten, aufweist und

ein Steuergerät in Verbindung mit dem wenigstens einen Sensorwandler, wobei das Steuergerät wenigstens einen Prozessor und eine Speichervorrichtung umfasst, die dazu ausgelegt ist, Anweisungen zu speichern, wobei die Anweisungen, wenn sie ausgeführt werden, bewirken, dass das Steuergerät:

die Lichtquelle so steuert, dass sie wenigstens eine erste Wellenlänge und eine zweite Wellenlänge eines Nah-Infrarot-Lichtes in einen Gewebekörper überträgt, wobei sich die erste Wellenlänge von der zweiten Wellenlänge unterscheidet,

den wenigstens einen Photodetektor so steuert, dass er den Gewebekörper für das Nah-Infrarot-Licht abtastet und Signale erzeugt, die repräsentativ für das abgetastete Nah-Infrarot-Licht sind, wobei die erzeugten für das abgetastete Nah-Infrarot-Licht repräsentativen Signale erste Signale, die für die erste Wellenlänge des Nah-Infrarot-Lichtes repräsentativ sind und eine AC-Komponente und eine DC-Komponente aufweisen, und zweite Signale, die für die zweite Wellenlänge des Nah-Infrarot-Lichtes repräsentativ sind und eine AC-Komponente und eine DC-Komponente aufweisen, umfassen,

die ersten Signale verarbeitet, um eine AC-Komponente der ersten Signale zu isolieren, wobei das Verarbeiten der ersten Signale zum Isolieren der AC-Komponente der ersten Signale das Filtern der ersten Signale, um die DC-Komponente der ersten Signale zu entfernen, umfasst,

eine Amplitude der AC-Komponente der ersten Signale ermittelt, wobei das Ermitteln der Amplitude der AC-Komponente der ersten Signale das Ermitteln eines Spitze-Tal-Wertes der AC-Komponente der ersten Signale umfasst, eine AC-Komponente eines ersten Gewebesauerstoffparameters unter Verwendung der Signale ermittelt, wobei es sich bei dem ersten Gewebesauerstoffparameter um Oxyhämoglobin ($HbO_2$) handelt, wobei das Ermitteln der AC-Komponente des ersten Gewebesauerstoffparameters den ermittelten Spitze-Tal-Wert der AC-Komponente der ersten Signale verwendet,

die zweiten Signale verarbeitet, um eine AC-Komponente der zweiten Signale zu isolieren, wobei das Verarbeiten der zweiten Signale zum Isolieren der AC-Komponente der zweiten Signale das Filtern der zweiten Signale, um die DC-Komponente der zweiten Signale zu entfernen, umfasst, eine Amplitude der AC-Komponente der zweiten Signale ermittelt, wobei das Ermitteln der Amplitude der AC-Komponente der zweiten Signale das Ermitteln eines Spitze-Tal-Wertes der AC-Komponente der zweiten Signale umfasst, eine AC-Komponente eines zweiten Gewebesauerstoffparameters unter Verwendung der Signale ermittelt, wobei es sich bei dem zweiten Gewebesauerstoffparameter um Desoxyhämoglobin (Hb) handelt, wobei der Schritt des Ermittelns der AC-Komponente des zweiten Gewebesauerstoffparameters den ermittelten Spitze-Tal-Wert der AC-Komponente der zweiten Signale verwendet, und

einen Wert der arteriellen Sauerstoffsättigung in Gewebe des Gewebekörpers unter Verwendung der ermittelten AC-Komponente des ersten Gewebesauerstoffparameters und der ermittelten AC-Komponente des zweiten Gewebesauerstoffparameters ermittelt, wobei der Schritt des Ermittelns der arteriellen Sauerstoffsättigung in Gewebe ein Verhältnis der ermittelten AC-Komponente des ersten Gewebesauerstoffparameters zu einer Summe aus der ermittelten AC-Komponente des ersten Gewebesauerstoffparameters und der ermittelten AC-Komponente des zweiten Gewebesauerstoffparameters verwendet.

7. Einrichtung nach Anspruch 6, wobei das Ermitteln des Spitze-Tal-Wertes der AC-Komponente der ersten Signale das Filtern der AC-Komponente der ersten Signale umfasst, um einen Wert zu ermitteln, der repräsentativ für den Spitze-Tal-Wert der AC-Komponente der ersten Signale ist, und wobei das Ermitteln des Spitze-Tal-Wertes der AC-Komponente der zweiten Signale das Filtern der AC-Komponente der zweiten Signale umfasst, um einen Wert zu ermitteln, der repräsentativ für den Spitze-Tal-Wert der AC-Komponente der zweiten Signale ist.

8. Einrichtung nach Anspruch 6, wobei das Ermitteln der AC-Komponente des ersten Gewebesauerstoffparameters das Ermitteln eines Spitze-Tal-Wertes der AC-Komponente des ersten Gewebesauerstoffparameters umfasst und wobei das Ermitteln der AC-Komponente des zweiten Gewebesauerstoffparameters des Gewebes das Ermitteln eines Spitze-Tal-Wertes der AC-Komponente des zweiten Gewebesauerstoffparameters des Gewebes umfasst.

9. Einrichtung nach Anspruch 6, wobei der wenigstens eine Detektor des wenigstens einen Sensorwandlers wenigstens einen nahen Detektor und wenigstens einen entfernten Detektor umfasst, wobei sich der wenigstens eine nahe Detektor in einem ersten Abstand zu der Lichtquelle befindet und sich der wenigstens eine entfernte Detektor in einem zweiten Abstand zu der Lichtquelle befindet und der zweite Abstand größer als der erste Abstand ist.

10. Einrichtung nach Anspruch 6, wobei der wenigstens eine Sensorwandler dazu ausgelegt ist, den Gewebekörper auf eine solche Weise zu empfangen, dass der Gewebekörper zwischen der Lichtquelle und dem Photodetektor angeordnet ist und das übertragene Nah-Infrarot-Licht von der Lichtquelle durch den Gewebekörper in einer zu dem Photodetektor verlaufenden Richtung übertragen wird.

**11.** Nichtflüchtiges computerlesbares Medium, das Computerprogrammanweisungen enthält, wobei die Computerprogrammanweisungen durch den wenigstens einen Computerprozessor ausführbar sind, um das Verfahren des nichtinvasiven Ermittelns eines Wertes der arteriellen Sauerstoffsättigung in Gewebe eines Gewebekörpers durchzuführen, wobei das Verfahren umfasst:

Steuern einer Lichtquelle so, dass sie wenigstens eine erste Wellenlänge und eine zweite Wellenlänge eines Nah-Infrarot-Lichtes in einen Gewebekörper überträgt, wobei sich die erste Wellenlänge von der zweiten Wellenlänge unterscheidet,
Steuern des wenigstens einen Photodetektors so, dass er den Gewebekörper für das Nah-Infrarot-Licht abtastet, und Erzeugen von Signalen, die repräsentativ für das abgetastete Nah-Infrarot-Licht sind, wobei die erzeugten für das abgetastete Nah-Infrarot-Licht repräsentativen Signale erste Signale, die für die erste Wellenlänge des Nah-Infrarot-Lichtes repräsentativ sind und eine AC-Komponente und eine DC-Komponente aufweisen, und zweite Signale, die für die zweite Wellenlänge des Nah-Infrarot-Lichtes repräsentativ sind und eine AC-Komponente und eine DC-Komponente aufweisen, umfassen,
Verarbeiten der ersten Signale, um eine AC-Komponente der ersten Signale zu isolieren, wobei der Schritt des Verarbeitens der ersten Signale zum Isolieren der AC-Komponente der ersten Signale das Filtern der ersten Signale, um die DC-Komponente der ersten Signale zu entfernen, umfasst,
Ermitteln einer Amplitude der AC-Komponente der ersten Signale, wobei der Schritt des Ermittelns einer Amplitude der AC-Komponente der ersten Signale das Ermitteln eines Spitze-Tal-Wertes der AC-Komponente der ersten Signale umfasst,
Ermitteln einer AC-Komponente eines ersten Gewebesauerstoffparameters unter Verwendung der Signale, wobei es sich bei dem ersten Gewebesauerstoffparameter um Oxyhämoglobin (HbO$_2$) handelt, wobei der Schritt des Ermittelns der AC-Komponente des ersten Gewebesauerstoffparameters des Gewebes den ermittelten Spitze-Tal-Wert der AC-Komponente der ersten Signale verwendet,
Verarbeiten der zweiten Signale, um eine AC-Komponente der zweiten Signale zu isolieren, wobei der Schritt des Verarbeitens der zweiten Signale zum Isolieren der AC-Komponente der zweiten Signale das Filtern der zweiten Signale, um die DC-Komponente der zweiten Signale zu entfernen, umfasst,
Ermitteln einer Amplitude der AC-Komponente der zweiten Signale, wobei der Schritt des Ermittelns einer Amplitude der AC-Komponente der zweiten Signale das Ermitteln eines Spitze-Tal-Wertes der AC-Komponente der zweiten Signale umfasst,
Ermitteln einer AC-Komponente eines zweiten Gewebesauerstoffparameters unter Verwendung der Signale, wobei es sich bei dem zweiten Gewebesauerstoffparameter um Desoxyhämoglobin (Hb) handelt, wobei der Schritt des Ermittelns der AC-Komponente des zweiten Gewebesauerstoffparameters den ermittelten Spitze-Tal-Wert der AC-Komponente der zweiten Signale verwendet, und Ermitteln eines Wertes der arteriellen Sauerstoffsättigung in Gewebe eines Gewebekörpers unter Verwendung der ermittelten AC-Komponente des ersten Gewebesauerstoffparameters und der ermittelten AC-Komponente des zweiten Gewebesauerstoffparameters, wobei der Schritt des Ermittelns der arteriellen Sauerstoffsättigung in Gewebe ein Verhältnis der ermittelten AC-Komponente des ersten Gewebesauerstoffparameters zu einer Summe aus der ermittelten AC-Komponente des ersten Gewebesauerstoffparameters und der ermittelten AC-Komponente des zweiten Gewebesauerstoffparameters verwendet.

## Revendications

**1.** Procédé mis en œuvre par ordinateur de détermination de manière non invasive d'une valeur de saturation en oxygène artériel tissulaire d'un corps tissulaire, comprenant :

la transmission d'au moins une première longueur d'onde et une seconde longueur d'onde de lumière proche infrarouge dans un corps tissulaire, la première longueur d'onde étant différente de la seconde longueur d'onde ;
la détection du corps tissulaire pour la lumière proche infrarouge et la production de signaux représentatifs de la lumière proche infrarouge détectée, les signaux produits représentatifs de la lumière proche infrarouge détectée comprenant des premiers signaux représentatifs de la première longueur d'onde de la lumière proche infrarouge, les premiers signaux ayant une composante CA et une composante CC, et des seconds signaux représentatifs de la seconde longueur d'onde de la lumière proche infrarouge, les seconds signaux ayant une composante CA et une composante CC ;

le traitement des premiers signaux pour isoler une composante CA des premiers signaux, l'étape de traitement des premiers signaux pour isoler la composante CA des premiers signaux comprenant le filtrage des premiers signaux pour éliminer la composante CC des premiers signaux ;

la détermination d'une amplitude de la composante CA des premiers signaux, l'étape de détermination d'une amplitude de la composante CA des premiers signaux comprenant la détermination d'une amplitude crête à crête de la composante CA des premiers signaux ;

la détermination d'une composante CA d'un premier paramètre d'oxygène tissulaire à l'aide des signaux, le premier paramètre d'oxygène tissulaire étant l'oxyhémoglobine ($HbO_2$), l'étape de détermination de la composante CA du premier paramètre d'oxygène tissulaire du tissu utilisant l'amplitude crête à crête déterminée de la composante CA des premiers signaux ;

le traitement des seconds signaux pour isoler une composante CA des seconds signaux, l'étape de traitement des seconds signaux pour isoler la composante CA des seconds signaux comprenant le filtrage des seconds signaux pour éliminer la composante CC des seconds signaux ;

la détermination d'une amplitude de la composante CA des seconds signaux, l'étape de détermination d'une amplitude de la composante CA des seconds signaux comprenant la détermination d'une amplitude crête à crête de la composante CA des seconds signaux ;

la détermination d'une composante CA d'un second paramètre d'oxygène tissulaire à l'aide des signaux, le second paramètre d'oxygène tissulaire étant la désoxyhémoglobine (Hb), l'étape de détermination de la composante CA du second paramètre d'oxygène tissulaire utilisant l'amplitude crête à crête déterminée de la composante CA des seconds signaux ; et

la détermination d'une valeur de saturation en oxygène artériel tissulaire d'un corps tissulaire à l'aide de la composante CA déterminée du premier paramètre d'oxygène tissulaire et de la composante CA déterminée du second paramètre d'oxygène tissulaire, l'étape de détermination de la saturation en oxygène artériel tissulaire utilisant un rapport de la composante CA déterminée du premier paramètre d'oxygène tissulaire et une somme de la composante CA déterminée du premier paramètre d'oxygène tissulaire et de la composante CA déterminée du second paramètre d'oxygène tissulaire.

2. Procédé selon la revendication 1, l'étape de détermination d'une amplitude crête à crête de la composante CA des premiers signaux comprenant le filtrage de la composante CA des premiers signaux pour déterminer une valeur représentative de l'amplitude crête à crête de la composante CA des premiers signaux ; et
l'étape de détermination d'une amplitude crête à crête de la composante CA des seconds signaux comprenant le filtrage de la composante CA des seconds signaux pour déterminer une valeur représentative de l'amplitude crête à crête de la composante CA des seconds signaux.

3. Procédé selon la revendication 1, l'étape de détermination de la composante CA du premier paramètre d'oxygène tissulaire comprenant la détermination d'une amplitude crête à crête de la composante CA du premier paramètre d'oxygène tissulaire ; et
l'étape de détermination de la composante CA du second paramètre d'oxygène tissulaire comprenant la détermination d'une amplitude crête à crête de la composante CA du second paramètre d'oxygène tissulaire.

4. Procédé selon la revendication 1, ladite au moins première longueur d'onde et ladite seconde longueur d'onde étant transmises en utilisant une source de lumière provenant d'un transducteur de capteur ayant au moins un détecteur proche et au moins un détecteur éloigné, ledit au moins un détecteur proche étant situé à une première distance de la source de lumière et ledit au moins un détecteur éloigné étant situé à une seconde distance de la source de lumière et la seconde distance étant supérieure à la première distance ; et
l'étape de détection utilisant l'au moins un détecteur proche et l'au moins un détecteur éloigné.

5. Procédé selon la revendication 1, l'étape de transmission et l'étape de détection utilisant un transducteur de capteur ayant une source de lumière et un détecteur de lumière, le transducteur de capteur étant configuré pour recevoir le corps tissulaire d'une manière telle que le corps tissulaire est disposé entre la source de lumière et le détecteur de lumière et la lumière infrarouge proche transmise est transmise depuis la source de lumière, à travers le corps tissulaire dans une direction vers le détecteur de lumière.

6. Appareil de détermination de manière non invasive d'une valeur de saturation en oxygène artériel tissulaire d'un corps tissulaire, comprenant :

au moins un transducteur de capteur comportant une source de lumière configurée pour produire au moins une première longueur d'onde et une seconde longueur d'onde de lumière proche infrarouge, et au moins un détecteur de lumière configuré pour détecter l'au moins ladite première longueur d'onde et ladite seconde longueur d'onde de lumière proche infrarouge ; et

un dispositif de commande en communication avec l'au moins un transducteur de capteur, le dispositif de commande comportant au moins un processeur et un dispositif de mémoire configuré pour stocker des instructions, les instructions stockées lorsqu'elles sont exécutées amènent le dispositif de commande à :

commander la source lumineuse pour transmettre au moins une première longueur d'onde et une seconde longueur d'onde de lumière proche infrarouge dans un corps tissulaire, la première longueur d'onde étant différente de la seconde longueur d'onde ;

commander l'au moins un détecteur de lumière pour détecter la lumière proche infrarouge dans le corps tissulaire et produire des signaux représentatifs de la lumière proche infrarouge détectée, les signaux représentatifs de la lumière proche infrarouge détectée comprenant des premiers signaux représentatifs de la première longueur d'onde de la lumière proche infrarouge, les premiers signaux ayant une composante CA et une composante CC, et des seconds signaux représentatifs de la seconde longueur d'onde de la lumière proche infrarouge, les seconds signaux ayant une composante CA et une composante CC ;

traiter les premiers signaux pour isoler une composante CA des premiers signaux, le traitement des premiers signaux pour isoler la composante CA des premiers signaux comprenant le filtrage des premiers signaux pour éliminer la composante CC des premiers signaux ;

déterminer une amplitude de la composante CA des premiers signaux, la détermination de l'amplitude de la composante CA des premiers signaux comprenant la détermination d'une amplitude crête à crête de la composante CA des premiers signaux ;

déterminer une composante CA d'un premier paramètre d'oxygène tissulaire à l'aide des signaux, le premier paramètre d'oxygène tissulaire étant l'oxyhémoglobine ($HbO_2$), la détermination de la composante CA du premier paramètre d'oxygène tissulaire utilisant l'amplitude crête à crête déterminée de la composante CA des premiers signaux ;

traiter les seconds signaux pour isoler une composante CA des seconds signaux, le traitement des seconds signaux pour isoler la composante CA des seconds signaux comprenant le filtrage des seconds signaux pour éliminer la composante CC des seconds signaux ;

déterminer une amplitude de la composante CA des seconds signaux, la détermination de l'amplitude de la composante CA des seconds signaux comprenant la détermination d'une amplitude crête à crête de la composante CA des seconds signaux ;

déterminer une composante CA d'un second paramètre d'oxygène tissulaire à l'aide des signaux, le second paramètre d'oxygène tissulaire étant la désoxyhémoglobine (Hb), l'étape de détermination de la composante CA du second paramètre d'oxygène tissulaire utilisant l'amplitude crête à crête déterminée de la composante CA des seconds signaux ; et

déterminer une valeur de saturation en oxygène artériel tissulaire du corps tissulaire en utilisant la composante CA déterminée du premier paramètre d'oxygène tissulaire et la composante CA déterminée du second paramètre d'oxygène tissulaire, l'étape de détermination de la saturation en oxygène artériel tissulaire utilisant un rapport de la composante CA déterminée du premier paramètre d'oxygène tissulaire et une somme de la composante CA déterminée du premier paramètre d'oxygène tissulaire et de la composante CA déterminée du second paramètre d'oxygène tissulaire.

7.  Appareil selon la revendication 6, la détermination de l'amplitude crête à crête de la composante CA des premiers signaux comprenant le filtrage de la composante CA des premiers signaux pour déterminer une valeur représentative de l'amplitude crête à crête de la composante CA des premiers signaux ; et
la détermination de l'amplitude crête à crête de la composante CA des seconds signaux comprenant le filtrage de la composante CA des seconds signaux pour déterminer une valeur représentative de l'amplitude crête à crête de la composante CA des seconds signaux.

8.  Appareil selon la revendication 6, la détermination de la composante CA du premier paramètre d'oxygène tissulaire comprenant la détermination d'une amplitude crête à crête de la composante CA du premier paramètre d'oxygène tissulaire ; et
la détermination de la composante CA du second paramètre d'oxygène tissulaire du tissu comprenant la détermination d'une amplitude crête à crête de la composante CA du second paramètre d'oxygène tissulaire du tissu.

9.  Appareil selon la revendication 6, l'au moins un détecteur de l'au moins un transducteur de capteur comprenant au moins un détecteur proche et au moins un détecteur éloigné, l'au moins un détecteur proche étant situé à une première distance de la source de lumière et l'au moins un détecteur éloigné étant situé à une seconde distance de la source de lumière et la seconde distance étant supérieure à la première distance.

10. Appareil selon la revendication 6, l'au moins un transducteur de capteur étant configuré pour recevoir le corps tissulaire d'une manière telle que le corps tissulaire est disposé entre la source de lumière et le détecteur de lumière et la lumière proche infrarouge transmise est transmise depuis la source de lumière, à travers le corps tissulaire dans une direction vers le détecteur de lumière.

11. Support lisible par ordinateur non transitoire contenant des instructions de programme informatique, les instructions de programme informatique étant exécutables par l'au moins un processeur informatique pour réaliser un procédé de détermination de manière non invasive d'une valeur de saturation en oxygène artérielle tissulaire d'un corps tissulaire, le procédé comprenant :

la commande d'une source lumineuse pour transmettre au moins une première longueur d'onde et une seconde longueur d'onde de lumière proche infrarouge dans un corps tissulaire, la première longueur d'onde étant différente de la seconde longueur d'onde ;
la commande d'au moins un détecteur de lumière pour détecter la lumière proche infrarouge dans le corps tissulaire et la production de signaux représentatifs de la lumière proche infrarouge détectée, les signaux représentatifs de la lumière proche infrarouge détectée comprenant des premiers signaux représentatifs de la première longueur d'onde de la lumière proche infrarouge, les premiers signaux ayant une composante CA et une composante CC, et des seconds signaux représentatifs de la seconde longueur d'onde de la lumière proche infrarouge, les seconds signaux ayant une composante CA et une composante CC ;
le traitement des premiers signaux pour isoler une composante CA des premiers signaux, l'étape de traitement des premiers signaux pour isoler la composante CA des premiers signaux comprenant le filtrage des premiers signaux pour éliminer la composante CC des premiers signaux ;
la détermination d'une amplitude de la composante CA des premiers signaux, l'étape de détermination d'une amplitude de la composante CA des premiers signaux comprenant la détermination d'une amplitude crête à crête de la composante CA des premiers signaux ;
la détermination d'une composante CA d'un premier paramètre d'oxygène tissulaire à l'aide des signaux, le premier paramètre d'oxygène tissulaire étant l'oxyhémoglobine (HbO$_2$), l'étape de détermination de la composante CA du premier paramètre d'oxygène tissulaire du tissu utilisant l'amplitude crête à crête déterminée de la composante CA des premiers signaux ;
le traitement des seconds signaux pour isoler une composante CA des seconds signaux, l'étape de traitement des seconds signaux pour isoler la composante CA des seconds signaux comprenant le filtrage des seconds signaux pour éliminer la composante CC des seconds signaux ;
la détermination d'une amplitude de la composante CA des seconds signaux, l'étape de détermination d'une amplitude de la composante CA des seconds signaux comprenant la détermination d'une amplitude crête à crête de la composante CA des seconds signaux ;
la détermination d'une composante CA d'un second paramètre d'oxygène tissulaire à l'aide des signaux, le second paramètre d'oxygène tissulaire étant la désoxyhémoglobine (Hb), l'étape de détermination de la composante CA du second paramètre d'oxygène tissulaire utilisant l'amplitude crête à crête déterminée de la composante CA des seconds signaux ; et
la détermination d'une valeur de saturation en oxygène artériel tissulaire d'un corps tissulaire à l'aide de la composante CA déterminée du premier paramètre d'oxygène tissulaire et de la composante CA déterminée du second paramètre d'oxygène tissulaire, l'étape de détermination de la saturation en oxygène artériel tissulaire utilisant un rapport de la composante CA déterminée du premier paramètre d'oxygène tissulaire et une somme de la composante CA déterminée du premier paramètre d'oxygène tissulaire et de la composante CA déterminée du second paramètre d'oxygène tissulaire.

*FIG. 1*

*FIG. 2*

Light source,
20

D1

D2

Cable,
24

Near detector,
22,22A

Far detector,
22,22B

*FIG. 3A*

Attachment
skin side

20

22,22A

22,22B

14

Sensor side view

24

18

*FIG. 3B*

120

114

122

*FIG. 4*

Pulsatile
Brain
(BW) →

Scalp →
(SW)

→ rTHb
Algorithm →

rTHb
rHbO2
rHb

→ High Pass Filter
HR Tunable
Sharp Cutoff | Low
Pass
Filter →

Peak To Peak
rTHb
rHbO2
rHb →

SaO2 =
rHbO2(pp) /
rTHb(pp) → Brain
SaO2

Pulsatile
Scalp →
(SW)

→ rTHb
Algorithm(2) →

rTHb
rHbO2
rHb

→ High Pass Filter
HR Tunable
Sharp Cutoff | Low
Pass
Filter →

Peak To Peak
rTHb
rHbO2
rHb →

SaO2 =
rHbO2(pp) /
rTHb(pp) → Scalp
SaO2

*FIG. 5*

*FIG. 6*

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63178120
- US 2019374140 A1
- US 20140171761 A
- US 8428674 B
- US 7072701 B
- US 8396526 B
- US 8788004 B
- US 10918321 B

### Non-patent literature cited in the description

- **ITO et al.** Arterial fraction of cerebral blood volume in humans measured by positron emission tomography. *Ann Nucl Med*, April 2001, vol. 15 (2), 111-6
- **PANG CC**. Measurement of body venous tone. *J Pharmacol Toxicol Methods*, 2000, vol. 44 (2), 341-60
- A novel method for the measurement of oxygen saturation in arterial blood. **ASHOKA REDDY K et al.** 2011 IEEE INTERNATIONAL INSTRUMENTATION AND MEASUREMENT TECHNOLOGY CONFERENCE (I2MTC 2011) : HANGZHOU, CHINA, 10 - 12 MAY 2011. IEEE, 10 May 2011, 1-5